# EUROPEAN PATENT SPECIFICATION

(11) **EP 2 862 576 B1**
(45) Date of publication and mention of the grant of the patent: **14.12.2016**
(21) Application number: 13382407.8
(22) Date of filing: 16.10.2013
(51) Int. Cl.: A61K 35/74, A61P 31/04, C12N 1/20

(54) **Microorganisms and compositions comprising them for use in the treatment or prevention of mastitis**
Mikroorganismen und Zusammensetzungen damit zur Verwendung bei der Behandlung oder Vorbeugung von Mastitis
Micro-organismes et compositions les comprenant à utiliser dans le traitement ou la prévention de la mastite

(43) Date of publication of application: 22.04.2015
(73) Proprietor: Casen Recordati, S.L., 50180 Utebo (Zaragoza) (ES)
(72) Inventor: Bello Navarro, Marta, 50180 UTEBO (ES); Tabuenca Navarro, Daniel, 50180 UTEBO (ES); Rodríguez Gómez, Juan Miguel, 28770 COLMENAR VIEJO (ES); Fernández Álvarez, Leónides, 28049 MADRID (ES); Jiménez Quintana, Esther Antonia, 28223 POZUELO DE ALARCÓN (ES)
(74) Representative: ZBM Patents - Zea, Barlocci & Markvardsen

(56) References cited:
- EP-A1- 1 997 499
- WO-A1-2005/034970
- TORRIANI S ET AL: "Differentiation of Lactobacillus plantarum, L. pentosus, and L. paraplantarum by recA gene sequence analysis and multiplex PCR assay with recA gene-derived primers", APPLIED AND ENVIRONMENTAL MICROBIOLOGY, AMERICAN SOCIETY FOR MICROBIOLOGY, US, vol. 67, no. 8, 1 August 2001 (2001-08-01) , pages 3450-3454, XP002261762, ISSN: 0099-2240, DOI: 10.1128/AEM.67.8.3450-3454.2001
- REBECA ARROYO ET AL: "Treatment of Infectious Mastitis during Lactation: Antibiotics versus Oral Administration of Lactobacilli Isolated from Breast Milk", CLINICAL INFECTIOUS DISEASES, vol. 50, no. 12, 15 June 2010 (2010-06-15) , pages 1551-1558, XP055078978, ISSN: 1058-4838, DOI: 10.1086/652763

## Description

The present invention relates to new approaches for preventing and treating mastitis in animals, including human. The invention also focuses on new isolated *Lactobacillus* strains and to compositions comprising them.

### BACKGROUND ART

Mastitis is the inflammation of breast tissue, namely of one or more lobules of the mammary gland, is a common disease during lactation since its incidence oscillates between 3 and 33 % of lactating mothers. Lactational mastitis usually has an infectious origin. Traditionally, *Staphylococcus aureus* is the most common etiological organism responsible, but *Staphylococcus epidermidis* and streptococci are occasionally isolated as well, being *Staphylococcus epidermidis* an emerging cause in human and veterinary medicine.

Mastitis typically develops when the milk is not properly removed from the breast. Milk stasis can lead to the milk ducts in the breasts becoming blocked, as the breast milk not being properly and regularly expressed. In human, it has also been suggested that blocked milk ducts can occur as a result of pressure on the breast, such as tight-fitting clothing or an over-restrictive bra, although there is sparse evidence for this supposition. Mastitis may occur when the baby is not appropriately attached to the breast while feeding, when the baby has infrequent feeds or has problems suckling the milk out of the breast.

The term mastitis varies depending on the geographic region. Outside the US it is commonly used for puerperal and nonpuerperal cases, in the US the term nonpuerperal mastitis is rarely used and alternative names such as duct ectasia, subareolar abscess and plasma cell mastitis are more frequently used. Puerperal mastitis is the inflammation of breast in connection with pregnancy, breastfeeding or weaning. The term nonpuerperal mastitis describes inflammatory lesions of the breast occurring unrelated to pregnancy and breastfeeding. Names for nonpuerperal mastitis are not used very consistently and include mastitis, subareolar abscess, duct ectasia, periductal inflammation, Zuska's disease and others.

Most common symptoms of mastitis include breast tenderness (warmth to the touch), feeling ill, swelling of breast, pain, redness, fever (38.3 °C or greater in human), flu-like symptoms including aches, shivering and chills, anxiety, stress, and fatigue.

Mastitis in animals is also an important issue, in particular in livestock where it is a concern because milk from the affected udders of livestock may enter the food supply and pose a health risk. In addition, it is a major condition in some species, like dairy cows, goat and sheep, having important economic impact on dairy industry. It is also of concern for public health.

Treatment of mastitis is performed by administering antibiotics for infections usually caused by bacteria of the skin or breastfed baby's mouth that enter the milk ducts through lesions or the opening of the nipples.

As commonly known in the art, the use of antibiotics implies several disadvantages, not only due to the low specificity of the treatment but also, and in part in consequence, in terms of side adverse effects. Thus, it is common that with wide-spectrum or Gram positive-targeted antibiotics be poorly effective to some infections caused by streptococcal and staphylococcal strains causing mastitis. Moreover, the treatment with antibiotics can cause the imbalance of the autochthonous microbiota, and can eliminate the commensal protective flora of the breast tissue or breast milk, as well as from intestines and other tissues, promoting colonization of some pathogens.

For these reasons, alternative actives and compositions have been studied in order to replace as much as possible the use of antibiotics. Among them, several probiotics have been tested for the treatment of mastitis in women.

As a way of example, it is mentioned the document of Arroyo et al., "Treatment of Infectious Mastitis during Lactation: Antibiotics versus Oral Administration of Lactobacilli Isolated from Breast Milk", Clinical Infectious Disease - 2010, Vol. No. 50(12), pp.: 1551-1558. This document discloses the use of two *Lactobacillus* strains, *Lactobacillus fermentum* (CECT 5716) and *Lactobacillus salivarius* (CECT 5713), both isolated from human breast milk, as a good alternative to antibiotics for treating mastitis. Nonetheless, as indicated by Arroyo the *Lactobacillus fermentum* (CECT 5716) produced flatulence in many of the treated women.

Another document wherein different *Lactobacillus* species are disclosed for the treatment of mastitis is the patent application EP1997499 (Puleva Biotech). This patent application discloses a process for isolating probiotic strains from mammal breast milk. The document also mentions, among others, some strains of *Lactobacillus plantarum* and *Lactobacillus fermentum* for use in the treatment of mastitis caused by *Staphylococcus aureus* in an animal model. Although mentioned for this use, no data are provided in a real human mastitis assay.

Although several efforts have been done in this field, there is thus still a need of alternative compounds and compositions for the treatment of mastitis, and in particular, of compounds that while having a therapeutic or prophylactic effect, they can be feasibly administered, and do not carry the side adverse effects of antibiotics.

### SUMMARY OF THE INVENTION

The inventors have found a strain of the species *Lactobacillus paraplantarum* that can be employed to ameliorate, prevent or treat all the symptomatology associated with mastitis in animals, including human. Surprisingly, when administered to human suffering from mastitis, the use of the strain of *Lactobacillus paraplantarum* allows a faster healing than that promoted by the use of antibiotics (narrow and wide spectrum antibiotics). In addition, the strain of *Lactobacillus paraplantarum* allows better and higher pathogen elimination from breast, at the same time the side adverse effects, associated and non-associated with the antibiotic administration, are minimized. According to the inventors' knowledge, this is the first time that a strain of *Lactobacillus paraplantarum* has been proved to be effective in mastitis.

Moreover, for the first time a strain of *Lactobacillus paraplantarum* has been isolated from human breast milk.

Although some *Lactobacillus plantarum* strains have been isolated from breast milk and faeces of mothers and babies, these correspond to a different species (Albesharat R et al. "Phenotypic and genotypic analyses of lactic acid bacteria in local fermented food, breast milk and faeces of mothers and their babies", Syst Appl Microbiol. - 2011 Vol. No. 34(2), pp.:148-55). The most frequently isolated strains pertain to the following species: *Lactobacillus salivarius, Lactobacillus gasseri,* and *Lactobacillus fermentum.*

Thus, in a first aspect the invention relates to an isolated *Lactobacillus paraplantarum* strain, deposited at the "Colección Española de Cultivos Tipo (CECT)" under the accession number CECT 8394, for use in the treatment and/or prevention of mastitis. The isolated strain for use in the treatment of mastitis is for use in any mammal, including humans.

This aspect can be formulated as the use of the isolated strain of *Lactobacillus paraplantarum,* deposited at the "Colección Española de Cultivos Tipo (CECT)" under the accession number CECT 8394, for the manufacture of a medicament, an edible or a nutritional composition for the treatment and/or prevention of mastitis in an animal, including a human. The present disclosure also relates to a method for the treatment or prevention of mastitis, comprising administering a therapeutically effective amount of the strain of *Lactobacillus paraplantarum,* deposited at the "Colección Española de Cultivos Tipo (CECT)" under the accession number CECT 8394,or a pharmaceutical composition, or a nutritional composition comprising at least that strain of *Lactobacillus paraplantarum,* together with pharmaceutically acceptable excipients or carriers, or edible ingredients in a subject or individual in need thereof, including a human.

Using compositions comprising mostly non-harmful microorganisms, such as *Lactobacillus,* many of them also usable as probiotics has the additional advantage of minimize the indiscriminate use of antibiotics. If the strains have further probiotic behavior, they can also be beneficial for animal (including human) gastro-intestinal tract.

Another aspect of the invention is then an isolated strain of *Lactobacillus paraplantarum* deposited at the "Colección Española de Cultivos Tipo (CECT)" under the accession number CECT 8394.

The strain of *Lactobacillus paraplantarum* of the invention, isolated from human breast milk of a woman from Spain was deposited, according to the Budapest Treaty, on the 3rd July 2013 in the "Colección Española de Cultivos Tipo (CECT)" in the Universidad de Valencia C.P 46980 Catedrático Agustín Escardino N° 9 Paterna, Valencia (Spain), by the depositor Laboratorios Casen Fleet S.L.U.(Autovía de Logroño, Km. 13.300 50180 Utebo (Zaragoza)- Spain). The strain of *Lactobacillus paraplantarum* was identified by the depositor with the reference LC29, and received the accession number CECT 8394. It was, in addition, declared viable.

As will be illustrated in the examples below, the strain of *Lactobacillus paraplantarum* CECT 8394 can also be used as a probiotic according to the FAO guidelines, which are the guidelines emitted by Food and Agriculture Organization of the United Nations. When administered to women suffering from mastitis, this strain was able to promote a faster healing than antibiotics commonly used for the treatment of mastitis. Women treated with the strain had less side adverse effects than women receiving antibiotics, and pain perception was also lower after treatment with the strain than with the antibiotics. Recurrences were also lower in women treated with *Lactobacillus paraplantarum* than in women receiving antibiotics, thus implying a preventive role of the strain. Thus, this strain of *Lactobacillus paraplantarum* CECT 8394 is for use in the treatment and/or prevention of mastitis in mammals, including humans.

In addition, the strain of *Lactobacillus paraplantarum* CECT 8394 provides the advantage of having an antimicrobial activity higher than other species of *Lactobacillus* also usable in the treatment of mastitis.

The strain of the invention can be supplied as an ingredient of a composition comprising carriers or other ingredients. It can also be in the form of a pure culture to be further combined (if required) with other compounds.

It is then a further aspect of the invention a bacterial pure culture which comprises the strain CECT 8394.

If supplied in the form of a composition, another aspect of the invention is a pharmaceutical composition comprising a therapeutically effective amount of the strain as defined above (*Lactobacillus paraplantarum* CECT 8394) as active ingredient, together with appropriate amounts of pharmaceutical acceptable excipients and/or carriers. These compositions can be used in the treatment and/or prevention of mastitis.

Another aspect of the invention is an edible product which comprises an effective amount of the strain as defined above *(Lactobacillus paraplantarum* CECT 8394) as active ingredient, together with appropriate amounts of other edible ingredients.

Either the composition (of any type) comprising the strain of *Lactobacillus paraplantarum* CECT 8394, or the pure culture of said strain can be in the form of a freeze-dried or lyophilized preparation.

Yet another aspect of the invention is a medical device comprising an effective amount of the strain as defined above. That is, an effective amount of the strain *Lactobacillus paraplantarum* CECT 8394, or a pure culture of the same.

As will be also illustrated in the examples, the strain of *Lactobacillus paraplantarum* CECT 8394 is a safe strain and displays probiotic properties. For this reason, it can be administered in the form of an edible product which comprises an effective amount of the strain of the invention (*Lactobacillus paraplantarum* CECT 8394)*,* together with appropriate amounts of other edible ingredients (i.e. compounds that can be safety eaten). Thus, another aspect of the invention is an edible product or composition which comprises an effective amount of the strain of the invention *(Lactobacillus paraplantarum* CECT 8394)*,* together with appropriate amounts of other edible ingredients. This edible product can be, in particular, a meal (yoghurt, snacks, meat, etc.) or a beverage, as indicated below.

### BRIEF DESCRIPTION OF THE DRAWINGS

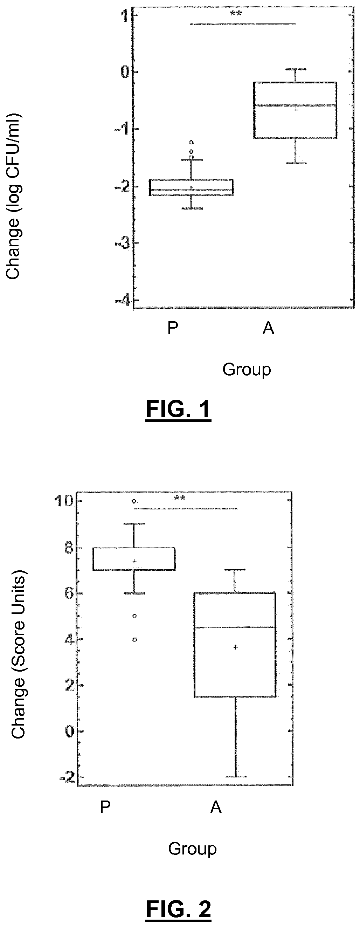

FIG. 1 is a graph showing, in the Y-axis, the changes in total bacterial count (Change, as the logarithm of the colony formation units per ml (log CFU/ml)) of breast milk samples of women with mastitis receiving the strain CECT 8394 (group P), and women receiving antibiotics (group A) treatment. Differences in the experienced changes were evaluated with Kruskall-Wallis test. The differences are shown in horizontal lines inside each graph (*P<0.05; **P<0.001).
FIG. 2 illustrates in the Y-axis for the same samples of FIG. 1, the changes in the breast pain score (score units) reported by women with mastitis receiving the strain CECT 8394 (group P), and women receiving antibiotics (group A) treatment. Breast pain score ranged from 0 (extremely painful) to 10 (no pain), and was evaluated by the one-way ANOVA test. The differences are shown in horizontal lines inside each graph (*P<0.05; **P<0.001).

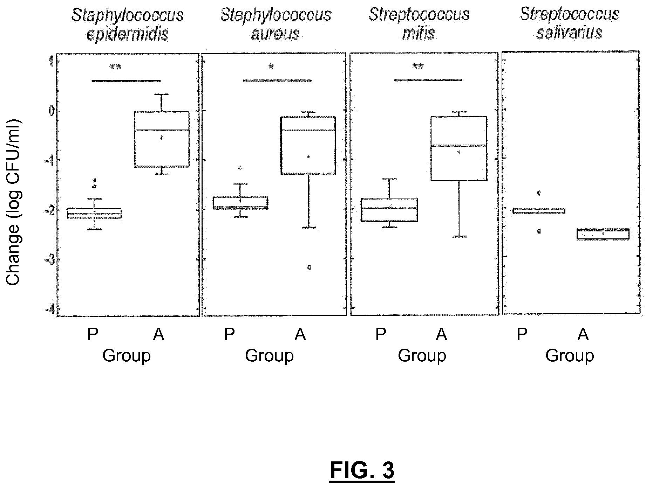

FIG. 3 is a graph with the changes of bacterial counts (Y-axis, Change (log CFU/ml)) of the species from the group of *Staphylococcus epidermidis, Staphylococcus aureus, Streptococcus mitis* and *Streptococcus salivarius* determined also in women of the P and A groups, as defined above. As in FIG. 1, differences in the changes experienced were evaluated with Kruskall-Wallis test for *Staphylococcus epidermidis* and *Streptococcus salivarius;* or one-way ANOVA for *Staphylococcus aureus* and *Streptococcus mitis,* and are shown in horizontal lines inside each graph (*P<0.05; **P<0.001).

### DETAILED DESCRIPTION OF THE INVENTION

All terms as used herein in this application, unless otherwise stated, shall be understood in their ordinary meaning as known in the art. Other more specific definitions for certain terms as used in the present application are as set forth below and are intended to apply uniformly throughout the specification and claims unless an otherwise expressly set out definition provides a broader definition. The following definitions are included for the purpose of understanding

In the sense of the invention the term "mastitis" encompasses puerperal and nonpuerperal mastitis, that is, inflammation of breast tissue due to any etiology, but in particular due to microbiological infections of said breast tissue. It also refers to duct ectasia, subareolar abscess, periductal inflammation, and Zuska's disease. Mastitis can be suffered by humans (men and women) as well as any other mammal, such as cows, goats, sheep, and horses.

The term "pharmaceutically acceptable" as used herein pertains to compounds, materials, compositions, and/or dosage forms which are, within the scope of sound medical judgment, suitable for use in contact with the tissues of a subject (e.g. human) without excessive toxicity, irritation, allergic response, or other problem or complication, commensurate with a reasonable benefit/risk ratio. Each carrier, excipient, etc., must also be "acceptable" in the sense of being compatible with the other ingredients of the formulation. Suitable carriers, excipients, etc. can be found in standard pharmaceutical texts, and include, as a way of example preservatives, agglutinants, humectants, emollients, and antioxidants.

The term "therapeutically effective amount" or "effective amount" as used herein, means an amount of an active agent (ingredient; i.e., a bacterial strain) high enough to deliver the desired benefit, either the treatment or prevention of the illness, but low enough to avoid serious side effects within the scope of medical judgment. The particular dose of compound administered according to this invention will of course be determined by the particular circumstances surrounding the case, including the compound administered, the route of administration, the particular condition being treated, and the similar considerations.

The term "food supplement" or "dietary supplement" are used herein as synonymous and relate to edible compositions intended to provide nutrients that may otherwise not be consumed in sufficient quantities. These supplements use to include, among others, vitamins, minerals, fatty acids, fiber and amino acids.

The term "medical device" means as defined by the European Directive 2007/47/EC any product, instrument, device, apparatus, computer program, material or article used in the medico sanitary field and being regulated by the European Directive 90/385/CEE of active implantable medical device, 98/79/CE of medical devices for in vitro diagnostic, and 93/42/CEE for general medical devices. The medical devices are included in the sanitary technologies. They are used in human beings for the diagnostic, prevention, control, treatment or alleviation of a disease, for the compensation of a deficiency; for investigating, substituting or modification of anatomy or a physiologic process; and for the regulation of contraception. Medical devices of the present invention relate to compositions or formulations ("medical device compositions"), or to products or devices comprising said compositions or formulations, comprising an effective amount of the strain as defined above (*Lactobacillus paraplantarum* CECT 8394) as active ingredient The term "medical device composition" is used herein to identify the compositions according to the invention, which compositions, as such (by their selves) are catalogued by the competent sanitary authorities as medical devices.

The term "probiotic" is to be understood in the sense of the present invention as live microorganisms which when administered in adequate amounts confer a health benefit on the host. The known benefits of enteral administration of probiotic microorganisms include enhanced host defense to disease improving the properties of the indigenous microbiota and increasing colonization resistance to the harmful microbiota. Probiotics have been suggested to play an important role in the formation or establishment of a well-balanced, intestinal microbiota in either infants or adults receiving high doses Of antibiotics. Lactic acid bacteria and bifidobacteria, especially specific strains of the genera *Lactobacillus, Streptococcus, Enterococcus* and *Bifidobacterium,* have been recommended for their use as probiotics.

Human breast milk is the particular source of the *Lactobacillus paraplantarum,* deposited at the "Colección Española de Cultivos Tipo (CECT)" under the accession number CECT 8394,. Other sources also encompassed with the present invention include the human breast skin, including mammary areolas and nipples.

As indicated, this is the first time a strain of *Lactobacillus paraplantarum* has been isolated from human breast milk.

The strain of *Lactobacillus paraplantarum* CECT 8394 may, as commonly done with microbiological products, be supplied in form of a bacterial pure culture of the strain. This culture, as well as any of the isolated bacteria from the same may be used as ingredient of a pharmaceutical composition, as well as an ingredient of any edible product, such as a food supplement.

In a particular embodiment, in any of the compositions or edible products comprising an effective amount of the strain as defined above, the strain is comprised from 10⁸ cfu/dose of composition or edible product to 10¹⁰ cfu/dose of composition or edible product. For the purposes of the invention, any ranges given include both the lower and the upper end-points of the range.Thus, effective amounts in particular embodiments are any multiples from 1 to 9 (1, 2, 3, 4, 5, 6, 7, 8, and 9) of 10⁸ cfu/dose, of 10⁹ cfu/dose and of 10¹⁰ cfu/dose. The amount indicated as cfu/dose relates to the colony forming units of the strain of the invention per dose of pharmaceutical composition or edible product (in particular food supplement).

In another particular embodiment, the "effective amount" of the strain of the invention is comprised from 10⁸ cfu/dose to 10¹⁰ cfu/dose, and it is administered or recommended in a dosage regimen from 2 to 3 doses containing the "effective amount" per day.

Therefore in a particular embodiment, the pharmaceutical compositions comprising a therapeutically effective amount of the strain as defined above *(Lactobacillus paraplantarum* CECT 8394*)* as active ingredient. In a particular embodiment, said therapeutically effective amount of the strain is comprised from 10⁸ cfu/dose of composition to 10¹⁰ cfu/dose of composition. In a particular embodiment, the pharmaceutical composition comprises a therapeutically effective amount of the strain consisting in *Lactobacillus paraplantarum* CECT 8394. In another particular embodiment, the pharmaceutical composition comprises a therapeutically effective amount from 10⁸ cfu/dose of composition to 10¹⁰ cfu/dose of composition of the strain consisting in *Lactobacillus paraplantarum* CECT 8394.

The pharmaceutical composition may be in the form of a solution, suspension, lyophilized compositions or other dried oral compositions (to be reconstituted with a liquid carrier or directly consumed), tablets, pills, capsules, etc. In a particular embodiment, the pharmaceutical composition is for oral administration. In another particular embodiment, the pharmaceutical composition can be formulated as a topical composition to be applied directly to the breast area with inflammation due to mastitis. The skilled in the art will select appropriate carriers and/ or excipients in order to formulate the pharmaceutical composition as desired. Other embodiments include compositions for vaginal, rectal or nasal route.

Indeed, the strain of *Lactobacillus paraplantarum* CECT 8394 can be an ingredient of any edible product or composition. In this edible product or composition, the strain of *Lactobacillus paraplantarum* will be in an effective amount to promote the desired effect. As above indicated the effective amount is in particular comprised from 10⁸ cfu/dose of edible product to 10¹⁰ cfu/dose of edible product. In a particular embodiment, the edible product is selected from the group consisting of a milk product, a yogurt, a curd, a cheese, a fermented milk, a powdered milk, a milk based fermented product, a meat based fermented product, an ice-cream, a cereal based fermented product, a beverage, a flour, a chewing-gum, a sweet, a sweet food, a pet food, a dietary or food supplement, a functional food, a clinical nutrition formula, or a nutritional complement. Dietary supplements intend to supply nutrients (vitamins, minerals, fatty acids or amino acids) that are missing or not consumed in sufficient quantity in a person's diet (infants, pregnant women, elderly people, etc.).

In a particular embodiment, the edible product is a food supplement comprising an effective amount of the strain as defined above (*Lactobacillus paraplantarum* CECT 8394*)* as active ingredient. In a particular embodiment, said effective amount of the strain is comprised from 10⁸ cfu/dose of composition to 10¹⁰ cfu/dose of composition. In another particular embodiment, the food supplement comprises an effective amount of the strain consisting in *Lactobacillus paraplantarum* CECT 8394. In another particular embodiment, the food supplement comprises an effective amount from 10⁸ cfu/dose of composition to 10¹⁰ cfu/dose of composition of the strain consisting in *Lactobacillus paraplantarum* CECT 8394.

Additionally, the strain as defined above, that is, the *Lactobacillus paraplantarum* CECT 8394, as well as pure cultures of the same, can also be used as a medical device once formulated as ingredient in a product considered by the authorities as a medical device or medical device composition. Thus, the strain as defined above can be applied directly or as an ingredient of a medical device. The medical device comprises an effective amount of the strain of the invention of the invention together with any acceptable excipient and/or carrier and/or any other components defining the medical device (i.e.: any support, or woven or non-woven fabric). In a particular embodiment, the medical device comprises an effective amount of the strain consisting in *Lactobacillus paraplantarum* CECT 8394.

A medical device allows the application or administration of the strain of the invention to a desired tissue surface, for example, in order to pharmaceutically and/or via the medical device itself treat or take care of the surface to which it is applied. In some embodiments, the composition comprising the strain of the invention is per se catalogued by the authorities as a medical device. In other particular embodiments of the medical devices, they correspond to sanitary articles that include woven or non-woven fabrics, in which the strain of the invention is homogeneously distributed along the surface of the fabric. This distribution can be performed, optionally, with the aid of a solvent. The solvent may also optionally be dried after layer or coating of the composition comprising the effective amount of the strain, is totally or partially distributed onto the surface of the fabric, thus obtaining fabrics with the properties conferred by the strain according to the invention.

In the particular case of the strain of *Lactobacillus paraplantarum* CECT 8394, it has been determined that it behaves probiotic properties. It is then a probiotic strain with the capacity of treating and/or preventing mastitis. As probiotic, the strain of *Lactobacillus paraplantarum* CECT 8394 is able to resist the environment of the gastrointestinal tract, adheres well to the gut epithelium (as demonstrated with the adhesion to Caco-2 cells and HT-29 cells), does not produce toxic compounds and competes with enteropathogens for the epithelium surface. Further, it has antimicrobial activity and is no toxic to animals (in terms of no translocation to spleen, liver and blood, and non-causing systemic infections).

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples and drawings are provided by way of illustration, and they are not intended to be limiting of the present invention. Furthermore, the present invention covers all possible combinations of particular and preferred embodiments described herein.

### EXAMPLES

### A) Isolation and taxonomic characterization of Lactobacillus paraplantarum CECT 8394

*Lactobacillus paraplantarum* CECT 8394 was isolated from human breast milk. Women complied with the following criteria: a) healthy women that had suffered from mastitis; b) normal pregnancy; and c) no perinatal problems in the mother or in the baby. Samples were collected in sterile tubes by means of manual pressing. Previously, an antiseptic (chlorhexidine) was applied in the areolas and the nipples. The samples were diluted in peptone and seeded in spiral in plates with different media.

*Lactobacillus paraplantarum* CECT 8394 was isolated from a Man Rogosa Sharpe (MRS) medium supplemented with L-cystein (MRS-Cys) incubated at 37 °C in anaerobiosis (85 % nitrogen, 10 % hydrogen and 5 % carbon dioxide) in a MACS-MG-1000-anaerobic workstation (DW Scientific, UK). The isolated strain was visualized by means of the microscope to analyse the morphology (rods in MRS white-cream colonies). Using Gram staining techniques, it was determined that the strain was Gram-positive, but negative for oxidase and catalase enzymatic activities.

The strain was identified at the specie level by amplification of the 16S RNA gene using the following primers:
Forward, pbl16: 5'-AGAGTTTGATCCTGGCTCAG-3' (SEQ ID NO: 1); and
Reverse, mlb16: 5'-GGCTGCTGGCACGTAGTTAG-3' (SEQ ID NO: 2).

PCR conditions were: 35 cycles of 96 °C, 30 seconds (s); 50 °C, 30 s; and 72 °C, 45 s; and a final extension step at 72 °C, 4 minutes (min).

Amplified fragment was purified with the NucleoSpin Extract II kit (Macherey-Nagel Gmb, Düren, Germany), and it was sequenced in an ABI 377A sequencer (Applied Biosystems, Foster City, USA). Obtained sequence was compared with the deposited in the EMBL database using BLAST algorithm (http://www.ncbi,nlm,nih.gov/BLAST). This fragment corresponded to SEQ ID NO: 3. This technology allowed classifying the strain to the *Lactobacillus plantarum-Lactobacillus paraplantarum- Lactobacillus pentosus* group. In order to determine in which specie the strain was emplaced, a specific multiplex PCR was performed. This allowed determining that the strain was from the *Lactobacillus paraplantarum* specie. The strain was also characterized by random amplification of polymorphic DNA (RAPD) in order to differentiate it from other strains of *Lactobacillus paraplantarum,* using genomic DNA from the MRS broth (10 ml) and the DNeasy tissue kit (Qiagen, Hilden, Germany). The primers for RAPD were:
Forward primer, ArgDei: 5'-ACCYTRGAAGGYGGYGATGTB-3' (SEQ ID NO: 4)
Reverse primer, OPL5: 5'-ACGCAGGCAC-3' (SEQ ID NO: 5)

### B) Gastrointestinal tract resistance of Lactobacillus paraplantarum CECT 8394

In order to investigate if the strain according to the invention could resist the gastric and intestine environment, 10⁹ cfu/ml in 25 ml of skimmed-milk were diluted in a buffer containing 6.2 g/l NaCl, 2.2 g/l KCI, 0.22 g/l CaCl₂ and 1.2 g/l NaHCO₃ to simulate saliva. Next, 5 ml of porcine gastric juice were added and the sample was incubated at 37 °C. A pH curve simulating the served as stomach pH after a yogurt ingestion was done (at the beginning pH 5.0, at 20 minutes pH 4.1, at 40 minutes pH 3.0, and at 60 minutes pH 2.1. Fractions were collected at 20, 40, 60 and 80 minutes. Fractions were adjusted to pH 6.5 (±2) with 1 M NaHCO₃ and then mixed with a solution containing 5 g/l Nacl, 0.6 g/l KCI, 0.3 g/l CaCl₂ , 4 % porcine bile and 7 % pancreatin (Sigma) to simulate duodenal juice. After 120 minutes the survival rate of the strain was determined seeding the samples in MRS plates (37 °C in anaerobiosis 48 h). Assays were performed in triplicate.

As can be deduced from Table 1, the viability of the strain to gastrointestinal tract conditions was high and similar to some commercial strains of other species.

**Table 1. Percentage of survival lactobacilli (10⁹ cfu/ml) in gastrointestinal tract conditions.**

| Strain | 0-20 min (pH 5.0) | 20-40 min (pH 4.1) | 40-60 min (pH 3.0) | 60-80 min (pH 2.1) | % Total |
|---|---|---|---|---|---|
| CECT 8394 (LC29) | 17.09±2.34 | 22.43±3.92 | 15.95±3.11 | 7.06±1.48 | 62.53 |
| *Lactobacillus fermentum* CECT 5716 | 9.71±0.54 | 13.75±1.75 | 20.91± | 10.00±0.01 | 54.37 |
| *Lactobacillus rhamnosus GG* | 14.83±2.04 | 2.03±1.28 | 11.45±0.41 | 12.69±0.59 | 41.00 |
| *Lactobacillus johnsonii La 1* | 8.58±0.85 | 11.53±1.21 | 11.87±0.42 | 9.61±1.04 | 41.59 |
| *Lactobacillus casei* imunitass | 20.79±0.51 | 25.68±3.77 | 16.69±6.38 | 0.077±0.04 | 63.24 |

### C) Caco-2/HT-29 adhesion assay

Adhesion of *Lactobacillus paraplantarum* CECT 8394 was evaluated in HT-29 and Caco-2 cells. Briefly, the cells were grown (30 min, 56 °C) in DMEM medium (PAA, Linz, Austria) with glucose (25 mM), sodium pyruvate (1 mM) supplemented with 10 % of bovine foetal serum (BPS), L-glutamine (2 mM), non-essential amino acids (1 %), penicillin (100 U/ml) and streptomycin (100 mg/ml).

HT-29 and Caco-2 cells were cultivated to confluence in 2 ml of the same medium but without antibiotics. Ten days later 1 ml of the medium was replaced by 1 ml of a bacterial suspension (10⁸ cfu/ml in DMEM). Inoculated cultures were incubated for 1 hour at 37 °C in 5 % CO2. Further, the monolayer was washed (5-times) with sterile PBS, fixed with methanol and Gram stained. Adhered lactobacilli in 20 random microscopic fields were counted (standard optical microscope).

The results are summarized in Table 2.

**Table 2: Number of adhered bacteria to HT-29 and Caco-2 cells.**

| Strain | HT-29 | Caco-2 |
|---|---|---|
| CECT 8394 (LC29) | 842.0±361.4 | 345.7±124.3 |
| *Lactobacillus fermentum* CECT 5716 | 905.65±596.6 | 277.01±185.4 |
| *Lactobacillus rhamnosus GG* | 820.2±350.4 | 361.6±138.9 |
| *Lactobacillus casei* imunitass | 164.2±88.7 | 17.2±11.3 |

Adhesion capacity to HT-29 and Caco-2 cells was noteworthy and similar to that of some commercial lactobacilli strains.

### D) Antimicrobial activity

The ability of the strain *Lactobacillus paraplantarum* CECT 8394 to inhibit growing of bacteria was assayed in front of *Enterococcus faecium* P21, *Enterococcus faecalis* TAB28, *Listeria monocytogenes* ScottA, *Listeria monocytogenes* Ohio, *Listeria innocua* RdC, *Staphylococcus aureus* CECT 5191, *Staphylococcus epidermidis* CECT 231, *Salmonella cholerasuis* CECT 4155, *Salmonella cholerasuis* CECT 409, *Salmonella cholerasuis* CECT 443, *Salmonella enteriditis* CECT 4396, *Escherichia coli* CECT 4076 (O157:H7), *Escherichia coli* RJM1, *Escherichia coli* RJM2, *Klebsiella pneumoniae* CECT 142, *Klebsiella oxytoca* CECT 860T and *Proteus vulgaris* CECT 484. Plates were incubated at 37 °C for 48 hours and the presence of inhibition zones around *Lactobacillus paraplantarum* CECT 8394 streaks were determined. Following Table 3 shows the antimicrobial activity.

The method for determining antimicrobial activity was performed according to the method previously described by Magnusson and Schnürer1 (Magnusson, J. et al., "Lactobacillus coryniformis subsp. coryniformis strain Si3 produces a broad-spectrum proteinaceous antifungal compound", Appl. Environ. Microbiol - 2001, Vol. No. 67, pp.:1-5) It was performed using MRS agar plates, on which *Lactobacillus paraplantarum* CECT 8394 was inoculated as approximately 2-cm-long lines and incubated at 32 °C for 48 h. The plates were then overlaid with the indicator microorganisms vehiculated in 10 ml of soft BHI agar (0.7%; Oxoid) media at an approximate concentration of 10⁴ cfu The plates overlaid with bacterial indicators were incubated at 32 or 37°C (depending on the optimal temperature) for 48 h. Finally, the plates were examined for clear zones of inhibition (> 2 mm) around the *Lactobacillus paraplantarum* CECT 8394 streaks. All experiments assaying inhibitory activity were performed in triplicate.

**Table 3. Antimicrobial activity of Lactobacillus paraplantarum CECT 8394**

| Indicator | CECT 8394 |
|---|---|
| *Enterococcus faecium* P21 | ++ |
| *Enterococcus faecalis* TAB28 | ++ |
| *Listeria monocytogenes* ScottA | +++ |
| *Listeria monocytogenes* Ohio | +++ |
| *Listeria innocua* RdC | +++ |
| *Salmonella cholerasuis* CECT 4155 | +++ |
| *Staphylococcus epidermidis* CECT 231 | +++ |
| *Salmonella cholerasuis* CECT 4155 | +++ |
| *Salmonella cholerasuis* CECT 409 | +++ |
| *Salmonella cholerasuis* CECT 443 | +++ |
| *Salmonella enteriditis* CECT 4396 | +++ |
| *Escherichia coli* CECT 4076 (O157:H7) | +++ |
| *Escherichia coli* RJM1 | +++ |
| *Escherichia coli* RJM2 | +++ |
| *Klebsiella pneumoniae* CECT 142 | ++ |
| *Klebsiella oxytoca* CECT 860T | ++ |
| *Proteus vulgaris* CECT 484 | ++ |

| | |
|---|---|
| +: inhibition halo <3mm; ++: inhibition halo 3-6 mm; +++: inhibition halo > 6 mm | |

*Lactobacillus paraplantarum* CECT 8394 showed a noteworthy antimicrobial activity against all tested microorganisms, being in particular effective against *Staphylococcus, Listeria, Salmonella,* and *Escherichia coli.* In comparison with the strain *Lactobacillus plantarum* CECT 7262 of a different species and disclosed in the document EP1997499 (Puleva Biotech), the strain *Lactobacillus paraplantarum* CECT 8394 was in addition highly active against the hazardous enteropathogens *Listeria monocytogenes* ScottA, *Klebsiella oxytoca* CECT 860T, and *Staphylococcus epidermidis* CECT 231. Also in comparison with *Lactobacillus plantarum* CECT 7262, the strain of the invention had a greater activity against *Enterococcus faecium* P21 and *Listeria innocua* RdC.

In parallel it was determined that the toxicity was no due to the production of bacteriocins (assayed with supernatant of MRS culture) or reuterin (assayed with supernatant of MRS culture and using *L.reuteri* CECT 925 as positive control). On the other hand, the strain showed a high co-aggregation capacity with potential pathogens *(Staphylococcus spp, Salmonella spp, Klebsiella spp,* and *E. coli*), thus avoiding interaction of the latter with human epithelial cells.

### E) Toxicity assays in animal models.

The aim of this study was to assess the global safety of the probiotic strain *Lactobacillus paraplantarum* CECT 8394 prior to the use in humans. It was evaluated the potential acute and repeated dose (4 weeks) oral toxicity; and the potential translocation to blood and some organs.

Wistar male and females rats (Charles River Inc., Marget, Kent, UK) were acclimated for 7 days prior to study initiation with an evaluation of health status.

Acute (limit test) and repeated dose (4 weeks) studies were conducted in accordance with European Union guidelines (EC Council Regulation No. 440, 2008 a,b).

In the acute test, 24 rats (12 males, 12 females) were distributed into two groups of 6 males and 6 females each. After an overnight fast each rat received skim milk (500 µl) orally (Control), or a single dose of 1 x 10¹⁰ cfu of *Lactobacillus paraplantarum* CECT 8394 dissolved in 500 µl of skim milk (Test). Animals were checked for clinical signs and mortality twice a day. At the end, the rats were euthanized by CO₂ inhalation, exsanguinated and necropsied.

The repeated dose (4 weeks) test was conducted with 48 rats (24 males, 24 females) divided in four groups of 6 males and 6 females each (control group; treated group; satellite control group; and satellite treated group). Rats of control groups received a daily dose of skim milk, and treated groups received 1 x 10⁹ cfu of *Lactobacillus paraplantarum* CECT 8394 dissolved in 500 µl of skim milk orally once a day over 4 weeks. Animals were checked for clinical signs and mortality twice a day. Rats of control and treated groups (no satellite) were deprived of food for 18 h, weighed, euthanized by CO₂ inhalation, exsanguinated and necropsied on day 29. Rats of satellite groups (control and treated) were kept 14 days more, before euthanization, without treatment to detect delayed occurrence, or persistence of, or recovery from toxic effects.

In acute oral toxicity test, no abnormal signs were observed. All the animals survived and there were no statistical differences in body weights among groups. Similarly, no statistically differences in body weight gain, food and water consumption were noted. Thus, the treatment with 1 x 10¹⁰ cfu of *Lactobacillus paraplantarum* CECT 8394 did not affect weight, body weight gain or food and water consumption. Haematological and clinical chemistry parameters were also not significantly different among groups, and no treatment-related changes were noted.

In repeated dose test, no mortality was observed, as well as no treatment-related changes. There were also no statistical differences in body weights and body weights gain among groups, in haematological parameters, as well as in the weight of the necropsied organs. Thus, the treatment with 1 x 10⁹ cfu of *Lactobacillus paraplantarum* CECT 8394 (4 weeks) did not promote any type of adverse effect. After 14 days without treatment, no treatment-related changes were detected.

Finally, it was also determined the Total Liver Glutathione (GSH) concentration in order to assess changes in the antioxidant defence due to the probiotic treatment. Also there was determined the bacterial translocation in blood, liver or spleen.

GSH concentration was analysed by homogenizing the livers (in 7.5 % trichloroacetic acid solution, 3000 xg for 10 min at 4 °C) and measuring in the supernatants GSH by means of a colorimetric assay (OxisResearch, Portland, OR).

Bacterial translocation was assessed by culturing blood in MRS media at 37 °C for 48 h in anaerobic conditions. Tissue samples were homogenized in buffered peptone (1 g/ml) and 100 µl of the homogenates were cultured on MRS as previously mentioned by blood. The plates were checked for the presence of lactobacilli. Positive growth was defined by the presence of even a single colony.

No significant differences in liver GSH concentration were detected between controls and treated groups. This indicates that treatment with *Lactobacillus paraplantarum* CECT 8394 did not cause oxidative stress and is consistent with the absence of bacteraemia since no lactobacilli could be isolated from blood, liver or spleen of the rats. It suggested that the strain does not cause either local or systemic infections in rats.

### F) Effect on the treatment of mastitis in comparison with antibiotics.

105 women with mastitis symptomatology were included in an assay. Inclusion criteria were: inflammation and breast pain and a bacterial count in milk greater than 4 log₁₀ cfu/ml. None of the women had consumed commercial antibiotics during the assay. The protocol was approved by the Ethical Comitee of Clinical Investigation of Hospital Universitario San Carlos (Madrid). Volunteers were randomized in two groups: a probiotic group, P; and a control antibiotic group, A.

The assay was performed for 21 days and, during this period, women of group P (n=62) received three capsules per day containing approximately 9 log₁₀ cfu/ml of the strain CECT 8394 (also named LC29). The capsules included the amount of the strain in methylcellulose. Capsules were maintained during the entire assay between 4-8 °C. Women of antibiotic group (A) (n=43) received an antibiotic treatment as prescribed in their ambulatory care centres. Milk samples were collected at the beginning (day 0) and at the end (day 21). Also at these times women were asked about the breast pain grade from 0 (highly intensive pain) to 10 (absence of pain).

Samples were seeded in plates of Baird Parker, Columbia, Mac Conkey and Sabouraud Dextrose Chloramphenicol (BioMerieux, Marcy l'Etoile, France) to selectively isolate of the major agents causing infectious mastitis and, in parallel, in Man Rogosa Sharpe plates (Oxoid, Basingstoke, UK) supplemented with L-cystein (0.5 g/)l (MRS-Cys) for the isolation of lactobacilli. Plates were incubated at 37 °C for 48 hours in aerobiosis except those of MRS-Cys medium, which were incubated under anaerobiosis (85 % nitrogen, 10 % hydrogen and 5 % carbon dioxide) in a MACS-MG-1000-anaerobic workstation (DW Scientific, UK).

Isolated bacteria were identified by means of morphological tests and biochemical assays. Those from the S. *epidermidis* and *S.aureus* species were confirmed with a multiplex PCR based on dnaJ in a termocycler Icycler (BioRad laboratories, Richmond, CA) with the following primers:
Forward primer for genera Staphylococcus, J-StGen: 5'-TGGCCAAAAGAGACTATTATGA-3' (SEQ ID NO: 6);
Reverse primer for S. *aureus,* J-StAur: 5'-GGATCTCTTTGTCTGCCG-3' (SEQ ID NO: 7);
Reverse primer for *S.epidermidis,* J-StEpi: 5'-CCACCAAAGCCTTGACTT-3' (SEQ ID NO: 8)

Identification of Streptococcus was performed by amplification (488 bp) and partial sequencing of the *tuf* gene (encoding the elongation factor Tu) using the following primers:
Forward primer, TufStrep1: 5'-GAAGAATTGVTTGAATTGGTTGAA-3' (SEQ ID NO: 9)
Reverse primer TufStrepR: 5'-GGACGGTAGTTGTTGAAGAATGG-3' (SEQ ID NO: 10).

Identification of isolated *Streptococcus mitis* was confirmed by optochin sensitivity assays, bile solubility assays and latex agglutination test with the Slide Pneumo kit (Biomerieux).

Remaining isolated bacteria were identified by means of amplification of a variable region of the 16S rRNA gene, using the following primers:
Forward, pbl16: 5'-AGAGTTTGATCCTGGCTCAG-3' (SEQ ID NO: 1); and
Reverse, mlb16: 5'-GGCTGCTGGCACGTAGTTAG-3' (SEQ ID NO: 2).

The fragment was then purified using the NucleoSpin Extract II kit (Macherey-Nagel Gmb, Düren, Germany), and it was sequenced in an ABI 377A sequencer (Applied Biosystems, Foster City, USA). Obtained sequence was compared with the deposited in the EMBL database using BLAST algorithm (http://www.ncbi,nlm,nih.gov/BLAST).

As can be seen in Tables 4, 5 below, at day 0 the bacterial concentration mean in the milk samples was similar in both groups (aprox. 4.40 log₁₀ cfu/ml), meanwhile lactobacilli were not detectable in any of the samples. The pain grade was high. At day 21, the bacterial concentration mean in the probiotic group (P) was significantly lower than that of the antibiotic group (A). In addition, as depicted in Table 6, women of group P healed faster and had fewer recurrences than those of group A, a part of having no adverse side-effects. At the end of the assay the pain grade was significantly lower in the group receiving the strain CECT 8394 (LC29) than that of the group receiving antibiotics, as derivable from Table 5.

**Table 4. Bacterial counts from breast milk, expressed as the mean log₁₀ cfu/ml (95 % CI), and breast pain feeling score, from extremely painful [0] to no pain [10], at the beginning of the study (day 0) and at the end of the trial (day 21).**

| | Day 0 | | | | | Day 21 | | | | |
|---|---|---|---|---|---|---|---|---|---|---|
| | Group P (CECT 8394) | | Group A | | | Group P (CECT 8394) | | Group A | | |
| | N | Mean | N | Mean | P^{a} | N | Mean | N | Mean | P^{a} |
| Total bacteria | 56 | 4.97 | 42 | 5.00 | 0.161 | 55 | 2.95 | 36 | 4.32 | <0.001 |
| *Staphylococcus epidermidis* | 34 | 4.90 | 33 | 4.21 | 0.139 | 34 | 2.88 | 27 | 4.20 | <0.001 |
| *Staphylococcus aureus* | 10 | 4.83 | 10 | 4.71 | 0.383 | 9 | 3.01 | 7 | 4.39 | <0.001 |
| *Streptococcus* | 19 | 4.67 | 14 | 4.75 | 0.913 | 16 | 2.90 | 13 | 4.06 | <0.001 |
| *mitis* | | | | | | | | | | |
| *Streptococcus salivarius* | 6 | 4.52 | 3 | 4.23 | 0.241 | 4 | 2.80 | 0 | | |
| Breast pain feeling score | 53 | 1.06 | 42 | 1.07 | 0.878 | 55 | 8.4 | 36 | 5.39 | <0.001 |

| | | | | | | | | | | |
|---|---|---|---|---|---|---|---|---|---|---|
| a: Kruskall Wallis test LSD 95 %. | | | | | | | | | | |

**Table 5: Changes in bacterial counts of breast milk samples and changes in breast pain score reported by the participants after treatment with CECT 8394 or antibiotic**

| | Group P (CECT 8394) | | Group A | | |
|---|---|---|---|---|---|
| | N | Mean (95%Cl) | N | Mean (95%Cl) | P^{a} |
| Total bacteria | 55 | -2.02 | 36 | -0.66 | 0.000 |
| *Staphylococcus epidermidis* | 34 | -2.02 | 26 | -0.53 | <0.001 |
| *Staphylococcus aureus* | 9 | -1.82 | 9 | -0.93 | 0.0039 |
| *Streptococcus mitis* | 19 | -1.96 | 14 | -0.85 | 0.000 |
| *Streptococcus salivarius* | 6 | -2.09 | 3 | -2.53 | 0.052 |
| Breast pain feeling score | 55 | 7.42 | 36 | 4.31 | 0.001 |

| | | | | | |
|---|---|---|---|---|---|
| a: Kruskall-Wallis for *Staphylococcus epidermidis and Streptococcus salivarius; one-way ANOVA for Staphylococcus aureus, Streptococcus mitis* and Breast pain feeling score. | | | | | |

**Table 6: Additional outcomes of the study**

| | Group P (n=62) | Group A (n=43) | P^{a} value |
|---|---|---|---|
| No healing | 0 | 12 | |
| Recurrences (2-month follow up) | 5* | 11 | 0.014 |
| Abcess | 0 | 1 | |
| Weaning | 0 | 6 | |
| Side effects (Vaginal candidiasis) | 0 | 8 | |
| Abandon the study | 1 | 6 | 0.013 |

| | | | |
|---|---|---|---|
| A x² test. (*) Five women experienced one episode but they were treated successfully with CECT 8394 (depositor's reference LC29). | | | |

FIGs 1 to 3 illustrate also the results of this assay.

In FIG. 1 there are depicted the changes (Y-axis) in total bacterial count (as the logarithm of the colony formation units per ml (log CFU/ml)) of breast milk samples of women with mastitis receiving the strain CECT 8394 (group P), and women receiving antibiotics (group A) treatment. The differences are shown in horizontal lines inside each graph (*P<0.05; **P<0.001).

In FIG. 2 the changes in the breast pain score (Y-axis, score units) are reported by women with mastitis receiving the strain CECT 8394 (group P), and women receiving antibiotics (group A) treatment. Breast pain score ranged from 0 (extremely painful) to 10 (no pain). The differences are shown in horizontal lines inside each graph (*P<0.05; **P<0.001).

Finally, FIG. 3 illustrates the particular of bacterial counts (Y-axis) of the species from the group of *Staphylococcus epidermidis, Staphylococcus aureus, Streptococcus mitis* and *Streptococcus salivarius* determined also in women of the P and A groups as defined above. As in FIG. 1, differences are shown in horizontal lines inside each graph (*P<0.05; **P<0.001)

All these data as a whole show the advantages inherently associated to the strain *Lactobacillus paraplantarum* CECT 8394, which can be safety administered to mammals including humans for treating mastitis of any etiology. The use of a strain of *Lactobacillus paraplantarum* instead of any commonly used antibiotic avoids side adverse effects of antibiotics. It supposes, in addition, a complementary or alternative treatment or method of prevention of mastitis with probiotic strains.

As above exposed, other lactobacilli from different species have been tested for the treatment of mastitis in women. In particular, the *Lactobacillus fermentum* CECT 5716 and *Lactobacillus salivarius* CECT 5713 have been assayed by Arroyo et al. *(supra).*

In a parallel assay, Arroyo et al. determined that the change in breast pain score in the groups receiving the probiotics (one capsule with 9 log₁₀ cfu/ml of probiotic) was higher than that promoted by antibiotic administration (from 2.35 at day 0 to 8.68 at day 21 for *Lactobacillus fermentum* CECT 5716; from 2.16 at day 0 to 8.61 at day 21 for *Lactobacillus salivarius* CECT 5713; and from 2.01 at day 0 to 5.81 at day 21 with antibiotics). However, several of the women treated in particular with *Lactobacillus fermentum* CECT 5716 declared flatulence, an annoying adverse side effect.

Although satisfactory results are achieved with the strains assayed by Arroyo et al., the strain *Lactobacillus paraplantarum* CECT 8394 of the invention allowed also an interesting reduction in breast pain (from 1.06 at day 0 to 8.4 at day 21; and from 1.07 at day 0 to 5.39 at day 21 with antibiotics) in the same time interval. Thus, the strain of *Lactobacillus paraplantarum* CECT 8394 (9 log₁₀ cfu/ml, three capsules) is faster in terms of healing than other strains of different species, and it does not imply adverse side effects.

In addition, the change in the bacterial counts of *Staphylococcus epidermidis, Staphylococcus aureus,* and *Streptococcus mitis* determined in the milk, was greater with the administration of the three capsules of *Lactobacillus paraplantarum* CECT 8394 (9 log₁₀ cfu/ml per capsule) in respect of the antibiotic treatment. Also no adverse effects were detected when the three capsules/day were administered.

### G) Formulations comprising the strain of Lactobacillus paraplantarum CECT 8394 of the invention.

Following are illustrated two batch formulas comprising the strain CECT 8394 according to the invention:

**Formula 1 in capsules:**

| | Weight (grams)/batch | % | Weight (mg)/capsule |
|---|---|---|---|
| *Lactobacillus paraplantarum* CECT 8394 (with maltodextrin) | 390.00 | 6.452 | 10.00 |
| Maltodextrin | 5421.00 | 89.677 | 139.00 |
| Magnesium stearate | 234.00 | 3.871 | 6.00 |
| | 6045.00 | 100.00 | 155.00 |

**Formula 2:**

| Description | Weight(g)/unit | % | Weight (Kg) /batch |
|---|---|---|---|
| Maltodextrin | 1.995 | 99.74 | 2.60 |
| *Lactobacillus paraplantarum* CECT 8394 (with maltodextrin) | 0.005 | 0.26 | 0.01 |
| Final weight | 2.000 | 100.00 | 2.61 |

If the formulas are formulated as capsules, they include:
Capsules of different materials such as gelatine or hydroxypropylmethylcellulose (HPMC); excipients, such as maltodextrin, caking agents, liquefying agents, HPMC, magnesium stearate, colloidal silica magnesium stearate, and combinations thereof; and the *Lactobacillus paraplantarum* CECT 8394 in lyophilized form and which can be accompanied with different carriers (maltodextrin or milk derivatives), as well as cryoprotectant agents.

The capsules may be packed in appropriate blisters of different materials (aluminium, Polyvinylidene chloride- PVDC, Aclar®, or Aquaba®).

If the formulas are formulated as a powdered in dispensing envelope, they include:
Excipients, such as maltodextrin, caking agents, liquefying agents, HPMC, magnesium stearate, colloidal silica magnesium stearate, and combinations thereof; and the *Lactobacillus paraplantarum* CECT 8394 in lyophilized form and which can be accompanied with different carriers (maltodextrin or milk derivatives), as well as cryoprotectant agents. The mixture in appropriate particle size is then loaded into envelopes of different materials.

During the manufacturing processes, humidity is controlled, being always equal or lower than 30 %. In particular, the excipients are controlled for their humidity and the filling of the capsules, blisters and of the envelopes is performed, as usual, under an inert gas atmosphere (for example under nitrogen atmosphere).

These exemplified formulas are not limitative. Indeed, weight proportions between ingredients may change meanwhile in the final composition the strain is comprised from 10⁸ cfu/dose of composition (capsule or envelope) to 10¹⁰ cfu/dose of composition (capsule or envelope).

### REFERENCES CITED IN THE APPLICATION

- Arroyo et al., "Treatment of Infectious Mastitis during Lactation:
   Antibiotics versus Oral Administration of Lactobacilli Isolated from Breast Milk", Clinical Infectious Disease - 2010, Vol. No. 50(12), pp.: 1551-1558.
- EP1997499.
- Albesharat R et al. "Phenotypic and genotypic analyses of lactic acid bacteria in local fermented food, breast milk and faeces of mothers and their babies", Syst Appl Microbiol. - 2011 Vol. No. 34(2), pp.:148-55.
- Magnusson, J. et al., "Lactobacillus coryniformis subsp. coryniformis strain Si3 produces a broad-spectrum proteinaceous antifungal compound", Appl. Environ. Microbiol - 2001, Vol. No. 67, pp.:1-5.

## Claims

1. Isolated *Lactobacillus paraplantarum* deposited at the "Colección Española de Cultivos Tipo (CECT)" under the accession number CECT 8394, for use in the treatment and/or prevention of mastitis.

2. An isolated strain of *Lactobacillus paraplantarum* deposited at the "Colección Española de Cultivos Tipo (CECT)" under the accession number CECT 8394.

3. The isolated strain of claim 2, which consists in the *Lactobacillus paraplantarum* deposited at the "Colección Española de Cultivos Tipo (CECT)" under the accession number CECT 8394.

4. A bacterial pure culture which comprises the strain of claim 2.

5. A pharmaceutical composition comprising a therapeutically effective amount of the strain as defined in any of claims 2-3 or a pure culture as defined in claim 4 as active ingredient, together with appropriate amounts of pharmaceutical acceptable excipients and/or carriers.

6. The pharmaceutical composition according to claim 5, wherein the therapeutically effective amount of the strain as defined in any of claims 2-3 is comprised from 10⁸ cfu/dose of composition to 10¹⁰ cfu/dose of composition.

7. The pharmaceutical composition according to any of claims 5-6 which is for oral administration.

8. An edible product which comprises an effective amount of the strain as defined in any of claims 2-3 or a pure culture as defined in claim 4 as active ingredient, together with appropriate amounts of other edible ingredients.

9. The edible product according to claim 8, which is selected from the group consisting of a milk product, a yogurt, a curd, a cheese, a fermented milk, a powdered milk, a milk based fermented product, a meat based fermented product, an ice-cream, a cereal based fermented product, a beverage, a flour, a chewing-gum, a sweet, a sweet food, a pet food, a dietary or food supplement, a functional food, a clinical nutrition formula, a nutritional complement, a formula for the elderly and an infant formula.

10. The edible product according to any of claims 8-9, which is a food supplement comprising an effective amount of the strain as defined in any of claims 2-3 or a pure culture as defined in claim 4 as active ingredient, together with appropriate amounts of other edible ingredients.

11. The edible product according to claim 10, wherein the effective amount in the food supplement of the strain as defined in any of claims 2-3 is comprised from 10⁸ cfu/dose of food supplement to 10¹⁰ cfu/dose of the food supplement.

12. A medical device comprising an effective amount of the strain as defined in any of claims 2-3 or a pure culture as defined in claim 4 as active ingredient.

## Patentansprüche

1. Isolierter *Lactobacillus paraplantarum,* der bei der "Colección Española de Cultivos Tipo (CECT)" unter der Hinterlegungsnummer CECT 8394 hinterlegt ist, zur Verwendung in der Behandlung und/oder Prävention von Mastitis.

2. Ein isolierter Stamm von *Lactobacillus paraplantarum,* der bei der "Colección Española de Cultivos Tipo (CECT)" unter der Hinterlegungsnummer CECT 8394 hinterlegt ist.

3. Der isolierte Stamm des Anspruchs 2, welcher in dem bei der "Colección Española de Cultivos Tipo (CECT)" unter der Hinterlegungsnummer CECT 8394 hinterlegten *Lactobacillus paraplantarum* besteht.

4. Eine bakterielle reine Kultur, welche den Stamm des Anspruchs 2 umfasst.

5. Eine pharmazeutische Zusammensetzung umfassend eine therapeutisch wirksame Menge des Stammes wie in einem der Ansprüche 2-3 definiert oder eine reine Kultur wie in Anspruch 4 definiert als Wirkstoff, zusammen mit geeigneten Mengen von pharmazeutisch akzeptablen Hilfsstoffen oder Trägerstoffen.

6. Die pharmazeutische Zusammensetzung nach Anspruch 5, wobei die therapeutisch wirksame Menge des Stammes wie in einem der Ansprüche 2-3 definiert von 10⁸ KbE/Dose der Zusammensetzung bis 10¹⁰ KbE/Dose der Zusammensetzung reicht.

7. Die pharmazeutische Zusammensetzung nach einem der Ansprüche 5-6, welche zur oralen Verabreichung ist.

8. Ein essbares Produkt, welches eine wirksame Menge des Stammes wie in einem der Ansprüche 2-3 definiert oder eine reine Kultur wie in Anspruch 4 definiert als Wirkstoff, zusammen mit geeigneten Mengen von weiteren essbaren Inhaltsstoffen umfasst.

9. Das essbare Produkt nach Anspruch 8, welches ausgewählt ist aus der Gruppe bestehend aus einem Milchprodukt, einem Jogurt, einem Käsebruch, einer Käse, einer fermentierten Milch, einem Milchpulver, einem auf Milch basierten fermentierten Produkt, einem auf Fleisch basierten fermentierten Produkt, einem Speiseeis, einem auf Getreide basierten fermentierten Produkt, einem Getränk, einem Mehl, einer Kaugummi, einer Süßigkeit, einem süßen Lebensmittel, einem Tierfutter, einem Nahrung- bzw. Ernährungsergänzungsmittel, einem funktionellen Lebensmittel, einem klinischen Ernährungsmittel, einer Nahrungsergänzung, einem Ernährungsmittel für ältere Menschen und einer Säuglingsanfangsnahrung.

10. Das essbare Produkt nach einem der Ansprüche 8-9, welches ein Nahrungsergänzungsmittel umfassend eine wirksame Menge des Stammes wie in einem der Ansprüche 2-3 definiert oder eine reine Kultur wie in Anspruch 4 definiert als Wirkstoff, zusammen mit geeigneten Mengen von weiteren essbaren Inhaltsstoffen ist.

11. Das essbare Produkt nach Anspruch 10, wobei die wirksame Menge im Nahrungsergänzungsmittel des Stammes wie in einem der Ansprüche 2-3 definiert von 10⁸ KbE/Dose des Nahrungsergänzungsmittels bis 10¹⁰ KbE/Dose des Nahrungsergänzungsmittels reicht.

12. Eine medizinische Vorrichtung umfassend eine wirksame Menge des Stammes wie in einem der Ansprüche 2-3 definiert oder eine reine Kultur wie in Anspruch 4 definiert als Wirkstoff.

## Revendications

1. *Lactobacillus paraplantarum* isolé déposé à la "Colección Española de Cultivos Tipo (CECT)" sous le numéro de dépôt CECT 8394, pour l'utilisation dans le traitement et/ou prévention de la mammite.

2. Une souche isolée de *Lactobacillus paraplantarum* déposée à la "Colección Española de Cultivos Tipo (CECT)" sous le numéro de dépôt CECT 8394.

3. La souche isolée de la revendication 2, qui consiste en le *Lactobacillus paraplantarum* déposé à la "Colección Española de Cultivos Tipo (CECT)" sous le numéro de dépôt CECT 8394.

4. Une culture pure bactérienne qui comprend la souche de la revendication 2.

5. Une composition pharmaceutique comprenant une quantité thérapeutiquement efficace de la souche telle que définie dans l'une quelconque des revendications 2-3 ou une culture pure telle que définie dans la revendication 4 comme ingrédient actif, conjointement avec des quantités appropriées d'excipients et/ou véhicules pharmaceutiquement acceptables.

6. La composition pharmaceutique selon la revendication 5, dans laquelle la quantité thérapeutiquement efficace de la souche telle que définie dans l'une quelconque des revendications 2-3 est comprise de 10⁸ UFC/dose de composition à 10¹⁰ UFC/dose de composition.

7. La composition pharmaceutique selon l'une quelconque des revendications 5-6, qui est destinée à la administration per voie orale.

8. Un produit comestible qui comprend une quantité efficace de la souche telle que définie dans l'une quelconque des revendications 2-3 ou une culture pure telle que définie dans la revendication 4 comme ingrédient actif, conjointement avec des quantités appropriées d'autres ingrédients comestibles.

9. Le produit comestible selon la revendication 8, qui est choisi dans le groupe constitué d'un produit laitier, un yaourt, un caillé, un fromage, un lait fermenté, un lait en poudre, un produit fermenté à base de lait, un produit fermenté à base de viande, une crème glacée, un produit fermenté à base de céréale, une boisson, une farine, une gomme à mâcher, une sucrerie, un aliment sucré, un aliment pour animaux, un complément diététique ou alimentaire, un alicament, une formule nutritionnelle clinique, un complément nutritionnel, une formule pour les personnes âgées et une formule infantile.

10. Le produit comestible selon l'une quelconque des revendications 8-9, qui est un complément alimentaire comprenant une quantité efficace de la souche telle que définie dans l'une quelconque des revendications 2-3 ou une culture pure telle que définie dans la revendication 4 comme ingrédient actif, conjointement avec des quantités appropriées d'autres ingrédients comestibles.

11. Le produit comestible selon la revendication 10, dans lequel la quantité efficace dans le complément alimentaire de la souche telle que définie dans l'une quelconque des revendications 2-3 est comprise de 10⁸ UFC/dose de complément alimentaire à 10¹⁰ UFC/dose de complément alimentaire.

12. Un dispositif médical comprenant une quantité efficace de la souche telle que définie dans l'une quelconque des revendications 2-3 ou une culture pure telle que définie dans la revendication 4 comme ingrédient actif.


### SEQUENCE LISTING

<110> Laboratories Casen Fleet S.L.U.
<120> Microorganisms and compositions comprising them for use in the treatment or prevention of mastitis
<130> P2782EP00
<160> 10
<170> PatentIn version 3.5
<210> 1
   <211> 20
   <212> DNA
   <213> Artificial
<220>
   <223> Forward primer
<400> 1
   agagtttgat cctggctcag 20
<210> 2
   <211> 20
   <212> DNA
   <213> Artificial
<220>
   <223> Reverse primer
<400> 2
   ggctgctggc acgtagttag 20
<210> 3
   <211> 626
   <212> DNA
   <213> Lactobacillus paraplantarum
<400> 3 
gcagtcgaac gaactctggt aatgattggt gcttgcatca tgaattacat ttgagtgagt 60

ggcgaactgg tgagtaacac gtgggaaacc tgcccagaag cgggggataa cacctggaaa 120

cagatgctaa taccgcataa caacttggac cgcatggtcc gagtttgaaa gatggcttcg 180

gctatcactt ttggatggtc ccgcggcgta ttagctagat ggtgaggtaa cggctcacca 240

tggcaatgat acgtagccga cctgagaggg taatcggcca cattgggact gagacacggc 300

ccaaactcct acgggaggca gcagtaggga atcttccaca atggacgaaa gtctgatgga 360

gcaacgccgc gtgagtgaag aagggtttcg gctcgtaaaa ctctgttgtt aaagaagaac 420

atatctgaga gtaactgttc aggtattgac ggtatttaac cagaaagcca cggctaacta 480

cgtgccagca gccgcggtaa tacgtaggtg gcaagcgttg tccggattta ttgggcgtaa 540

agcgagcgca ggcggttttt taagtctgat gtgaaagcct tcggctcaac cgaagaagtg 600
 catcggaaac tgggaaactt gagtgc 626
<210> 4
   <211> 21
   <212> DNA
   <213> Artificial
<220>
   <223> Forward primer
<220>
   <221> misc_feature
   <222> (4)..(4)
   <223> y is t or c
<220>
   <221> misc_feature
   <222> (6)..(6)
   <223> r is g or a
<220>
   <221> misc_feature
   <222> (12)..(12)
   <223> y is t or c
<220>
   <221> misc_feature
   <222> (15)..(15)
   <223> y is t or c
<220>
   <221> misc_feature
   <222> (21) .. (21)
   <223> b is g or t or c
<400> 4
   accytrgaag gyggygatgt b 21
<210> 5
   <211> 10
   <212> DNA
   <213> Artificial
<220>
   <223> Reverse primer
<400> 5
   acgcaggcac 10
<210> 6
   <211> 22
   <212> DNA
   <213> Artificial
<220>
   <223> Forward primer
<400> 6
   tggccaaaag agactattat ga 22
<210> 7
   <211> 18
   <212> DNA
   <213> Artificial
<220>
   <223> Reverse primer
<400> 7
   ggatctcttt gtctgccg 18
<210> 8
   <211> 18
   <212> DNA
   <213> Artificial
<220>
   <223> Reverse primer
<400> 8
   ccaccaaagc cttgactt 18
<210> 9
   <211> 24
   <212> DNA
   <213> Artificial
<220>
   <223> Forward primer
<220>
   <221> misc_feature
   <222> (10)..(10)
   <223> v is a or g or c
<400> 9
   gaagaattgv ttgaattggt tgaa 24
<210> 10
   <211> 23
   <212> DNA
   <213> Artificial
<220>
   <223> Reverse primer
<400> 10
   ggacggtagt tgttgaagaa tgg 23